# EUROPEAN PATENT APPLICATION

(11) **EP 4 847 067 A1**
(43) Date of publication of application: **02.12.2026**
(21) Application number: 25744720.1
(22) Date of filing: 24.01.2025
(51) Int. Cl.: A61M 15/00

(54) **DOSE INDICATION DEVICE FOR INHALER**

(30) Priority: 25.01.2024 CN 202410107636
(71) Applicant: GUANGZHOU CHIA TAI INNOVATIVE PHARMACEUTICAL CO., LTD., Guangdong 510700 (CN)
(72) Inventor: ZENG, Lingyue, Guangzhou, Guangdong 510700 (CN); LIU, Wenbin, Guangzhou, Guangdong 510700 (CN); YANG, Zhenchang, Guangzhou, Guangdong 510700 (CN); ZENG, Qi, Guangzhou, Guangdong 510700 (CN)
(74) Representative: WSL Patentanwälte Partnerschaft mbB
(86) International application number: PCT/CN2025/074627
(87) International publication number: WO 2025/157257

(57) **Abstract**

The present disclosure provides a dose indication device for an inhaler, and belongs to the field of inhaler manufacturing technologies. The dose indication device includes an outer shell and a base connected to each other, and further includes an actuating assembly, a transmission assembly, and a dose indicator ring; a dose indication window and an opening are provided on a side wall of the outer shell, the actuating assembly includes an actuating portion and a push-pull portion connected to each other, and the actuating portion can penetrate through the opening and is movable laterally between a first position and a second position. When the actuating assembly moves between the first position and the second position, the transmission gear is driven to rotate, which in turn causes the dose indicator ring to rotate, thereby achieving dose indication. The dose indication device of the present disclosure can count and/or indicate the number of times the fluid container in the inhaler has been used or the remaining number of uses, and has the advantages of a simple structure, high fault tolerance rate, good versatility, low production cost, ease of assembly and testing, and the like.

## Description

### CROSS-REFERENCE TO RELATED APPLICATIONS

The present application claims priority and benefit to the Chinese Patent Application No. 202410107636.X filed with the National Intellectual Property Administration, PRC on January 25, 2024, which is incorporated herein by reference in its entirety.

### TECHNICAL FIELD

The present application relates to a dose indication device for an inhaler, and in particular, to a dose indication device for a soft mist inhaler, and belongs to the field of inhaler manufacturing technologies.

### BACKGROUND

An inhaler typically includes a nebulising assembly that pumps and atomizes a fluid for inhalation by a user after a replaceable fluid container is installed in the inhaler.

After installation of the fluid container, the user may not be aware of the usage of the fluid in the fluid container. When the fluid container stores a medicinal solution for treating a chronic or acute condition, in the case where the medicinal solution in the fluid container has reached or exceeded a predetermined number of uses, if the medicinal solution is continuously used, there is a high possibility that the ejection dose is unstable, causing a risk of medication. Therefore, there is an urgent need for a dose indication device that can display the number of times a fluid container has been used or the remaining number of uses.

Boehringer Ingelheim has developed and launched Respimat, a propellant-free soft mist inhaler. The earliest marketed Respimat is a single-use device, and subsequently, RESPIMAT re-usable was available. This product has a reusable version, in which a replaceable container containing fluid may be inserted into an upper housing of a nebulizer and closed by a lower housing; by rotating the lower housing, the container moves downward in the nebulizer, a driving spring may be under tension, and meanwhile, the fluid can be pumped into a pressure chamber of a pressure generator. After a button is manually pressed, the container moves upward, the driving spring is released and compresses the pressure chamber, such that the fluid in the pressure chamber is at a high pressure and is eventually atomized through a nozzle. A dose indication device is connected to the lower part of the container, and a driving portion at the bottom of the lower housing is designed to actuate the indicator device when the container moves relative to the driving portion, such that the indicator device can be configured to count and/or indicate the number of times the container has been used or the remaining number of uses. Since the actuation is achieved by the longitudinal movement of the dose indication device relative to the driving portion, strict control over the longitudinal dimensions of the entire device, particularly the distance of the above-mentioned relative movement, is required to ensure the actuation effect. Therefore, the respective components need to be precisely manufactured and assembled. However, containers with different sample sizes often have different heights. If these containers are adapted to the same inhaler, the longitudinal dimensions vary greatly, resulting in significant differences in actuation effects and poor versatility. In addition, the dose indication device needs to perform counting and display tests after assembly. Since an actuating assembly in the dose indication device is completely built-in, an additional power source is required to simulate the driving portion during the test process, and the relative positions of the driving portion and the actuating assembly need to be strictly controlled. Therefore, the test is also difficult and costly.

There are also some other types of dose indication devices in the prior art that are designed to achieve a dose indicating function by means of electronics, the use of which increases cost and may have battery life issues. In addition, these dose indication device structures are relatively complex, require a large number of small mechanical components, are costly, and are difficult to assemble.

### SUMMARY

In view of the shortcomings of the prior art, the present application provides a dose indication device for an inhaler. In one aspect, by providing an opening on a side wall of an outer shell, an assembly space of some components in the dose indication device is increased, which is beneficial to the assembly; meanwhile, an actuating portion is provided to penetrate through the opening and at least partially protrude outward from the opening, such that the actuating portion is partially externally disposed, which is beneficial to the test process, and even no additional driving portion needs to be provided, a tester may directly press the actuating portion manually and then observe whether the counting and indication are performed normally, thereby reducing the test difficulty and the test cost. In another aspect, an inner wall of a lower end of the housing gradually contracts inward along a central axis of the housing to form the driving portion, such that the component structure is simplified, and moreover, since the horizontal cross-section of the inner wall of the lower end of the housing is substantially circular, when the dose indication device is mounted on the fluid container, the actuating portion can be actuated by the driving portion as long as the actuating portion faces the inner wall, regardless of the specific direction, which facilitates the assembly of the dose indication device. In yet another aspect, the dose indication device provided by the present application is actuated by the relative lateral movement of the actuating portion and the driving portion, such that the whole device has high tolerance for the longitudinal dimensions, which reduces the production precision of the components, facilitates the assembly of the respective components, and is more conducive to adapting to fluid containers of different specifications. The fluid containers with different specifications often have different heights, and there may be slight longitudinal dimension differences between the same product from different batches. As long as the housing can accommodate the fluid container connected to the dose indication device and the actuating portion can move laterally between the first position and the second position, effective actuation can be achieved, and accurate counting can be achieved. However, there is a relatively low requirement for the precision of the longitudinal dimensions (e.g., the height of the fluid container, the height of the housing, the distance between the bottom of the housing and the bottom of the dose indication device, etc.) of the entire inhaler, thereby improving the tolerance of the inhaler for fluid containers of different batches, improving the adaptability of the inhaler to fluid containers of different specifications, and improving the versatility of the entire device.

In a first aspect, the present application provides a dose indication device for an inhaler, the inhaler including: a housing for receiving a fluid container and a nebulising assembly for nebulising a fluid, the fluid container being movable relative to the housing, and the housing including a driving portion for actuating the dose indication device; the dose indication device including:
an outer shell, through which the dose indication device is connected to the fluid container, and a dose indication window and an opening being provided on a side wall of the outer shell;
a base, connected to the outer shell, and a cavity being provided between the outer shell and the base;
an actuating assembly, slidably connected to the base, under actuation of the driving portion, the actuating assembly being laterally movable relative to the base between a first position and a second position, the actuating assembly including an actuating portion and a push-pull portion connected to each other, the actuating portion penetrating through the opening, when the actuating assembly is at the first position, the actuating portion at least partially protruding outward from the opening, and when the actuating assembly is at the second position, the actuating portion at least partially retracting into the cavity; the push-pull portion including a push-pull rod and being located in the cavity;
a transmission assembly, located in the cavity and including a transmission shaft and a transmission gear located on the transmission shaft, the transmission gear being arranged opposite to the push-pull rod, and the transmission gear being rotatable about the transmission shaft under an action of the push-pull rod; and
a dose indicator ring, coaxially arranged with the base and rotatable about a central axis of the base, graduations for displaying count values being provided on an outer periphery of the dose indicator ring, and the dose indicator ring being further provided with protruding portions capable of interacting with the transmission gear; and
when the actuating assembly moves between the first position and the second position, the push-pull rod driving the transmission gear to rotate, which in turn causes the dose indicator ring to rotate via the protruding portions, thereby achieving dose indication.

Further, the fluid container moves up and down relative to the housing such that the dose indication device moves up and down relative to the housing.

Further, a fluid container connecting portion for connecting the fluid container is provided at an end of the outer shell away from the base.

Further, the dose indication window and the opening are oppositely arranged.

Further, in order to improve the actuation stability of the dose indication device, the dose indication device includes at least two actuating assemblies, the at least two actuating assemblies being symmetrically arranged relative to a central axis of the outer shell. It can be understood that the number and positions of the openings match the number and positions of the actuating assemblies, and the openings do not affect the dose indication window.

Preferably, the dose indication device includes two actuating assemblies, two openings are provided on the side wall of the outer shell, and the two openings are located on two sides of the dose indication window, respectively. Further, in order to make full use of various component structures of the dose indication device and improve the aesthetics of the device, when the actuating assembly is at the second position, the actuating portion is configured to completely retract into the cavity.

Further, the push-pull portion further includes a connecting rod, and the push-pull rod is located at an end part of the connecting rod and is fixedly connected to the actuating portion through the other end part of the connecting rod.

Preferably, in order to improve the stability of the interaction between the push-pull rod and the transmission gear, the push-pull portion includes two connecting rods arranged in parallel, and the push-pull rod is arranged between the two connecting rods.

Further, a stop portion is provided on a side of the base facing the outer shell, an elastic assembly is provided between the actuating assembly and the stop portion, one end of the elastic assembly abuts against the stop portion, and the other end thereof abuts against the actuating assembly.

Preferably, the elastic assembly is a spring; further preferably, one end of the spring abuts against the stop portion, and the other end thereof abuts against a side of the actuating portion away from the housing.

Further, the driving portion is an inward protruding structure provided on an inner wall of the housing. Preferably, the driving portion is a separate component detachably or fixedly connected to the housing, and when the dose indication device moves relative to the housing, the driving portion enables the actuating assembly to move laterally between the first position and the second position.

Preferably, to simplify the structure, the driving portion is a part of the housing, and when the fluid container moves relative to the housing together with the dose indication device, the actuating portion of the actuating assembly comes into contact with the driving portion, and the driving portion actuates the actuating portion, such that the actuating assembly moves laterally between the first position and the second position.

Further preferably, an inner wall of a lower end of the housing gradually contracts inward along a central axis of the housing to form the driving portion, when the actuating assembly is at the first position, the actuating portion does not abut against the driving portion, and when the actuating assembly moves from the first position to the second position, the actuating portion abuts against the driving portion, and an acting force between the driving portion and the actuating portion causes the actuating portion to at least partially retract into the outer shell.

Still further preferably, the horizontal cross-section of the inner wall of the lower end of the housing is substantially circular, and the inner wall of the lower end of the housing gradually contracts circumferentially inward along the central axis of the housing to form the driving portion.

Further preferably, abutting portions of the driving portion and the actuating portion are matched smooth curved surfaces.

Preferably, at least two rib structures are provided on the inner wall of the housing, guide grooves for allowing the actuating portion to move are formed between the rib structures, and the guide grooves are offset from top to bottom in a direction toward a central axis of the housing.

Further preferably, 2-40 rib structures are evenly arranged on the inner wall of the housing; preferably, 2-30 rib structures are arranged; more preferably, 10-30 rib structures are arranged; and further preferably, 20-30 rib structures are arranged.

Further, in order to reduce the movement resistance, the bottom of the guide groove is a smooth curved surface, and the abutting surfaces between the actuating portion and the guide groove are matched smooth curved surfaces. Further, the dose indication device further includes transmission assembly brackets fixed to the base, and the transmission assembly is rotatably arranged in the transmission assembly brackets.

Further, the protruding portions are located at a top of the dose indicator ring and axially extend toward the transmission gear, the transmission gear includes a helical groove and radially protruding teeth, and during rotation of the transmission gear, at least one protruding portion engages in the helical groove all the time.

Further, the push-pull rod acts on the teeth to drive the transmission gear to rotate.

Further, the dose indication device further includes a lock pawl for preventing the transmission gear from rotating in reverse, the lock pawl being arranged within the cavity and extending toward the transmission gear.

Preferably, the lock pawl is arranged on the base.

Further preferably, the stop portion and the lock pawl are the same component.

Further, the dose indication device further includes a locking mechanism configured to prevent the actuating portion from moving laterally between the first position and the second position and/or to prevent the dose indicator ring from further rotating after a predetermined number of uses of the fluid container.

Preferably, the locking mechanism includes a first locking portion located on the actuating assembly and a second locking portion located on the dose indicator ring, the second locking portion engaging with the first locking portion after the predetermined number of uses of the fluid container, so as to prevent the actuating assembly from moving laterally between the first position and the second position.

Further preferably, the first locking portion is located in the cavity and extends toward the top of the dose indicator ring, the second locking portion is located at the top of the dose indicator ring and extends toward the base, and after the predetermined number of uses of the fluid container, the second locking portion engages with the first locking portion, so as to prevent the actuating assembly from moving laterally between the first position and the second position.

Further preferably, the locking mechanism prevents the actuating assembly from moving laterally after the predetermined number of uses of the fluid container.

Further preferably, the second locking portion is located on a path of lateral movement of the first locking portion, thereby preventing the actuating assembly from moving laterally from the first position to the second position. Further preferably, the locking mechanism prevents the actuating assembly from moving laterally from the second position to the first position after the predetermined number of uses of the fluid container.

Further, the dose indication device further includes an anti-inadvertent actuation mechanism for preventing the dose indication device from being inadvertently actuated prior to use of the fluid container.

Still further, the anti-inadvertent actuation mechanism is detachably connected to the actuating portion, and when the anti-inadvertent actuation mechanism is disengaged from the actuating portion, the actuating portion is in an actuatable state.

Preferably, the anti-inadvertent actuation mechanism includes protrusions, grooves matched with the protrusions are provided in a part of the actuating portion located outside the cavity, and prior to use of the fluid container, the anti-inadvertent actuation mechanism is connected to the grooves through the protrusions, such that the actuating portion is in a non-actuatable state.

Further preferably, the anti-inadvertent actuation mechanism includes two protrusions, and the actuating portion is correspondingly provided with two grooves.

In a second aspect, the present application provides a fluid container for an inhaler, a bottom of the fluid container being connected to the dose indication device described above.

Preferably, the fluid container is fixedly connected to the dose indication device, and when the fluid container needs to be replaced, the fluid container and the dose indication device are replaced together.

In a third aspect, the present application provides an inhaler, a housing of the inhaler receiving the fluid container connected to the dose indication device described above.

In a fourth aspect, the present application further provides a method for counting and/or indicating the number of times the fluid container in the inhaler has been used or the remaining number of uses by using the dose indication device described above.

In a fifth aspect, the present application further provides a dose indication device having the structure as described in the first aspect of the present application. It can be understood that the dose indication device may be used in other devices or equipment requiring counting and/or displaying, apart from an inhaler, as long as the device or equipment is provided with a driving portion capable of actuating the dose indication device. Similarly, the dose indication device may be connected to the fluid container through the outer shell, or may also be connected to other components in the device or equipment through the outer shell, or even the dose indication device may be present individually in the device or equipment, as long as the driving portion can actuate the dose indication device during motions requiring counting and/or displaying.

In summary, the present application provides a dose indication device, and a fluid container and an inhaler using the dose indication device. When the fluid container moves up and down within the housing, the dose indication device is actuated by utilizing the relative lateral movement of the actuating portion and the driving portion, such that the overall device has high tolerance for the longitudinal dimensions. For different products, or the same product from different batches, the height of the fluid container varies, the initial position of the assembled fluid container relative to the bottom of the housing varies, and/or the relative movement distance varies. The present application can convert the vertical movement of the fluid container into the lateral movement of the actuating portion in the horizontal direction, which in turn translates into rotational movement of the transmission gear by a certain angle, finally achieving a valid count. Compared with traditional products requiring precise control of the dimensions of the respective components, the present application increases the fault tolerance rate of the dose indication device and reduces the production cost. Moreover, the entire dose indication device has a simple structure and high stability, which is particularly beneficial for adapting to fluid containers of different specifications, thereby improving the versatility. In addition, the protruding design of the actuating portion not only facilitates assembly, but also facilitates a test process, thereby reducing the test difficulty and costs.

Further, in the present application, a locking mechanism is further provided, which can effectively and firmly lock the fluid container after a predetermined number of uses, thereby preventing the user from continuing to use the fluid container when the fluid is nearly depleted or has been depleted, improving the safety, and also reminding the user to replace the fluid container with a new one.

Still further, in the present application, an anti-inadvertent actuation mechanism is further provided, which avoids accidental actuation of the dose indication device prior to use of the fluid container and is beneficial for ensuring the accuracy of counting and indication.

### BRIEF DESCRIPTION OF THE DRAWINGS

To better understand the present application, the following describes the present application in detail with reference to the accompanying drawings. In the accompanying drawings:
FIG. 1 is a cross-sectional view of an inhaler in an initial state according to the present application;
FIG. 2 is a cross-sectional view of an inhaler in an actuated state according to the present application;
FIG. 3 is a cross-sectional view of a housing of an inhaler according to the present application;
FIG. 4 is a schematic exploded view of a dose indication device according to the present application;
FIG. 5 is a cross-sectional view of an assembled dose indication device according to the present application;
FIG. 6 is a sectional view of a dose indication device at a first position;
FIG. 7 is a sectional view of a dose indication device at a second position;
FIG. 8 is a sectional view of a dose indication device after a predetermined number of uses of a fluid container;
FIG. 9 is a sectional view of a dose indication device in a locked state;
FIG. 10 is a schematic view of a dose indicator ring;
FIG. 11 is a bottom view of a dose indicator ring;
FIG. 12 is a schematic diagram of a transmission assembly;
FIG. 13 is a schematic diagram of a dose indication device with an anti-inadvertent actuation mechanism;
FIG. 14 is a schematic diagram of an anti-inadvertent actuation mechanism according to the present application; and
FIG. 15 is a schematic diagram of an actuating assembly according to the present application.

Description of reference numerals:
100 dose indication device; 110 outer shell; 111 opening; 112 dose indication window; 113 fluid container connecting portion; 120 dose indicator ring; 121 protruding portion; 122 second locking portion; 130 actuating assembly; 131 actuating portion; 1311 groove; 132 push-pull portion; 1321 connecting rod; 1322 push-pull rod; 133 first locking portion; 140 transmission assembly; 141 transmission shaft; 142 transmission gear; 1421 helical groove; 1422 tooth; 150 elastic assembly; 160 base; 161 transmission assembly bracket; 162 stop portion; 163 lock pawl; 164 dose indicator ring bracket; 170 anti-inadvertent actuation mechanism; 171 protrusion; 200 housing; 201 driving portion; 300 fluid container; 400 nebulising assembly; 500 inhaler.

### DETAILED DESCRIPTION

In order to make the technical solutions of the present application and the obtained technical effects clearer, the following detailed description is made with reference to specific embodiments, but the embodiments of the present application are not limited thereto.

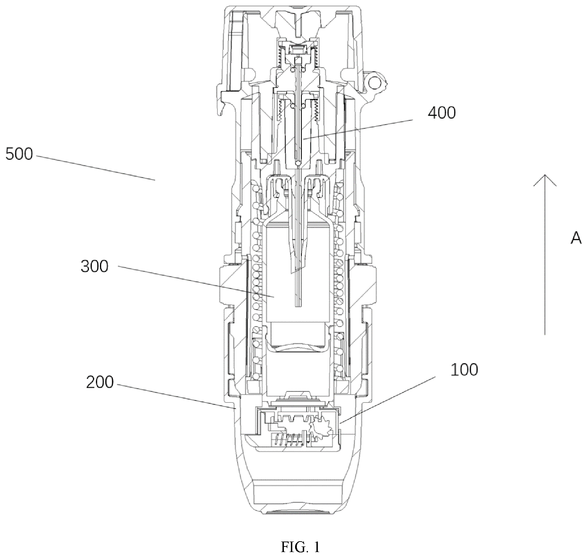

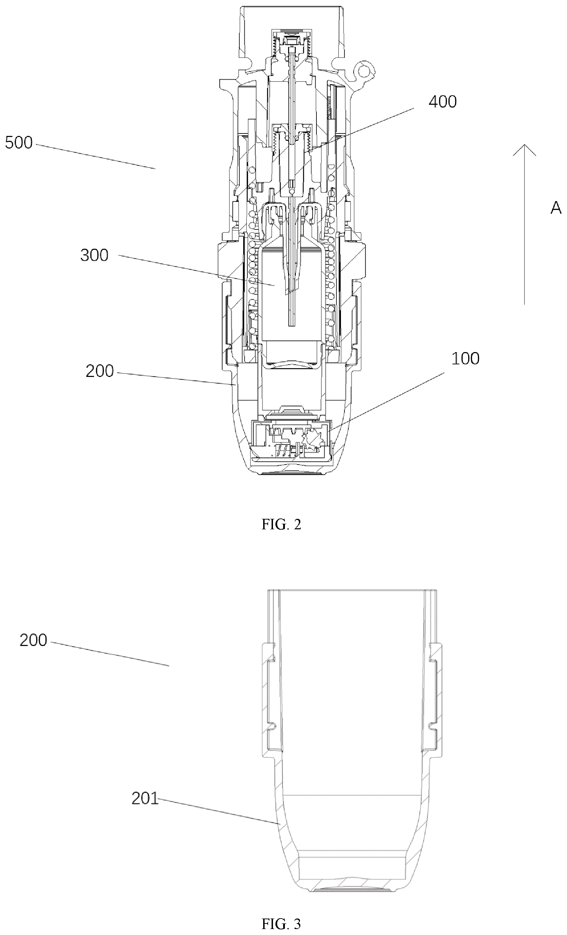

In the present application, the terms "upper and down", "vertical", or "longitudinal" refer to the direction extending from the base 160 to the fluid container connecting portion 113 (the direction where the central axis of the inhaler 500 is located, or the direction where the central axis of the dose indication device 100 is located). In addition, the direction indicated by arrow A in FIGs. 1 and 2 is defined as upward.

In the present application, the terms "horizontal", "lateral", or "left and right" are directions orthogonal to the "upper and down", "vertical", or "longitudinal" directions.

In the present application, the term "outward" refers to a direction pointing out of a cavity formed after the outer shell 110 and the base 160 are connected, or a direction pointing away from the central axis of the inhaler 500 (or the dose indication device 100), e.g., a direction pointing from the cavity to an inner wall of the housing 200 when the dose indication device 100 is located in the housing 200.

In the present application, the term "inward" refers to a reverse direction opposite to the outward direction described above.

In the present application, the term "first position" refers to the position of the actuating assembly 130 relative to the base 160 when the part of the actuating portion 131 protruding outward from the opening 111 is maximized. In this case, the driving portion 201 is disengaged from the actuating assembly 130, or the driving portion 201 is in mere contact with the actuating assembly 130, but no interaction occurs.

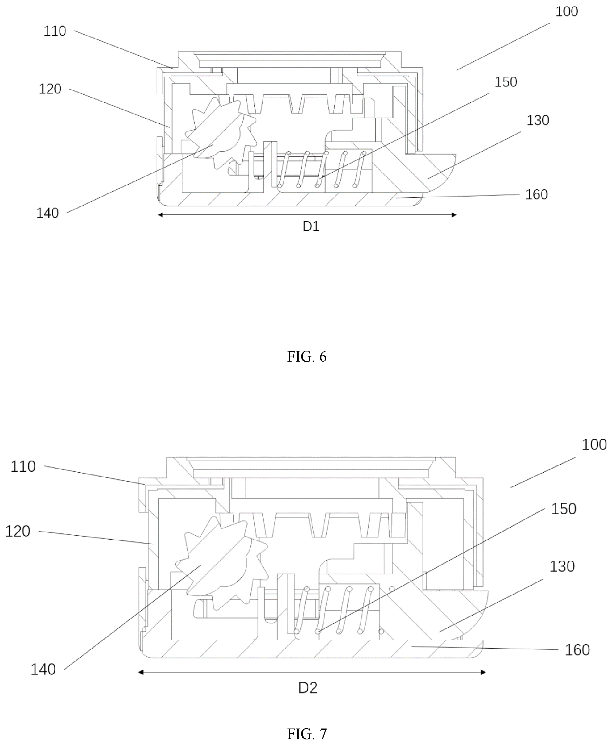

In the present application, the term "second position" refers to the position of the actuating assembly 130 relative to the base 160 when the part of the actuating portion 131 protruding outward from the opening 111 is minimized. In this case, the interaction force between the driving portion 201 and the actuating assembly 130 is also maximized. In the present application, the term "maximum width of the dose indication device" refers to the width of the dose indication device 100, as shown by D1 in FIG. 6, when the part of the actuating portion 131 protruding outward from the opening 111 is maximized.

In the present application, the term "minimum width of the dose indication device" refers to the width of the dose indication device 100, as shown by D2 in FIG. 7, when the part of the actuating portion 131 protruding outward from the opening 111 is minimized.

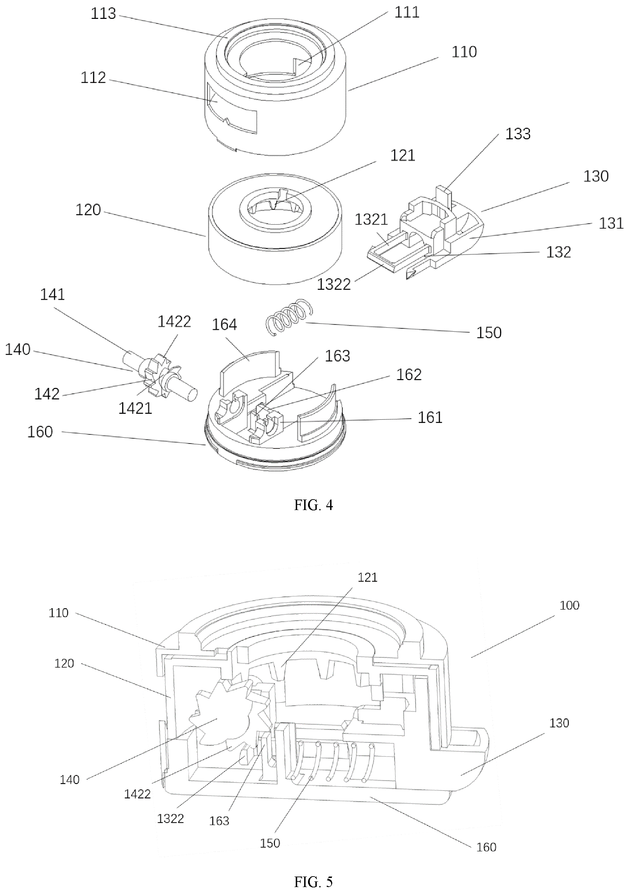

In the present application, the term "clockwise" or "counterclockwise" refers to the rotational direction of the transmission gear 142 as viewed from the angle shown in FIG. 5.

Unless defined otherwise, all technical and scientific terms used herein have the same meaning as commonly understood by those skilled in the art to which the present application belongs. The terms used herein in the specification of the present application are for the purpose of describing specific embodiments only and are not intended to limit the present application.

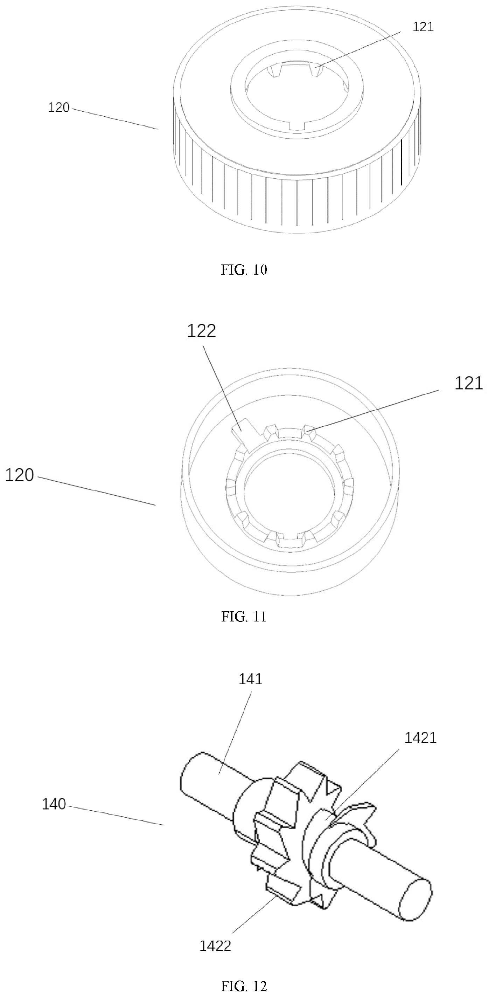

As shown in FIGs. 1-15, the present application provides a dose indication device 100. The dose indication device 100 is applicable to a reusable inhaler 500, and includes, but is not limited to, RESPIMAT^{®} re-usable by Boehringer Ingelheim (BI) or an inhalation device disclosed in Patent Application No. 201880027066.6.

Illustratively, as shown in FIGs. 1 and 2, the inhaler 500 includes a housing 200 for receiving a fluid container 300 (e.g., a medicine bottle) and a nebulising assembly 400 for nebulising the fluid. The fluid container 300 is movable relative to the housing 200. The housing 200 includes a driving portion 201. The fluid container 300 completes one longitudinal reciprocating motion in the inhaler 500 during a process of using the nebulising assembly 400 to complete one nebulization inhalation, and the driving portion 201 is configured to drive or actuate the dose indication device 100 when the fluid container 300 reciprocates. The structures of the inhaler 500, the nebulising assembly 400, and the fluid container 300 are the prior art, and details are not described herein again.

The present application provides a dose indication device 100 for an inhaler 500. As shown in FIGs. 1-5, the dose indication device includes an outer shell 110, through which the dose indication device 100 is connected to the fluid container 300, and a dose indication window 112 and an opening 111 being provided on a side wall of the outer shell 110. A base 160 is further included. The base 160 is connected to the outer shell 110, and a cavity is provided between the outer shell 110 and the base 160. In order to accommodate most inhalers 500 and fluid containers 300 on the market, the outer shell 110 is preferably cylindrical.

In this embodiment, the dose indication device 100 is connected to the bottom of the fluid container 300 through the outer shell 110. When the fluid container 300 moves up and down within the housing 200, the dose indication device 100 is also driven to move up and down within the housing 200.

In other embodiments of the present application, the dose indication device 100 is connected to other positions of the fluid container 300, such as the top, one side, or even the periphery surrounding the fluid container 300, through the outer shell 110. The objectives of the present application can be achieved as long as the dose indication device 100 can follow the relative movement between the fluid container 300 and the housing 200.

In an embodiment of the present application, the connection manner between the outer shell 110 and the fluid container 300 is a snap-fit connection. It can be understood that another connection manner, such as a direct connection or an indirect connection, or a fixed connection or a detachable connection, can achieve the objectives of the present application.

The dose indication device 100 provided in the present application further includes an actuating assembly 130 which is slidably connected to the base 160. Under the actuation of the driving portion 201, the actuating assembly 130 is laterally movable relative to the base 160 between a first position and a second position. The actuating assembly 130 includes an actuating portion 131 and a push-pull portion 132 connected to each other. The actuating portion 131 penetrates through the opening 111. When the actuating assembly 130 is at the first position, the actuating portion 131 at least partially protrudes outward from the opening 111, and when the actuating assembly 130 is at the second position, the actuating portion 131 at least partially retracts into the cavity. The push-pull portion 132 includes a push-pull rod 1322 and is located in the cavity.

In this embodiment, the actuating assembly 130 is connected to the base 160 via a sliding buckle. It can be understood by those skilled in the art that the actuating assembly 130 may be slidably connected to the base 160 in other manners as long as the actuating assembly 130 is allowed to move laterally on the base 160 while preventing the actuating assembly 130 from completely disengaging from the base 160.

In this embodiment, the actuating assembly 130 includes an actuating portion 131 and a push-pull portion 132 connected to each other. When the actuating portion 131 is actuated by the driving portion 201, the push-pull portion 132 may follow the actuating portion 131 to move laterally between the first position and the second position.

In a specific embodiment of the present application, the push-pull portion 132 further includes a connecting rod 1321, and the push-pull rod 1322 is located at end parts of the connecting rod 1321 and is fixedly connected to the actuating portion 131 through the other end parts of the connecting rod 1321. In other embodiments of the present application, the push-pull rod 1322 may be connected to the actuating portion 131 in other manners. It can be understood that a direct connection or an indirect connection, a fixed connection or a detachable connection, or the like, can achieve the objectives of the present application.

In an embodiment of the present application, as shown in FIG. 4, the push-pull portion 132 includes two connecting rods 1321 arranged in parallel, and the push-pull rod 1322 is arranged between the two connecting rods 1321, such that the stability of the interaction between the push-pull rod 1322 and the transmission gear 142 is improved.

The dose indication device 100 provided in the present application further includes a transmission assembly 140. The transmission assembly 140 is located in the cavity and includes a transmission shaft 141 and a transmission gear 142 located on the transmission shaft 141. The transmission gear 142 is arranged opposite to the push-pull rod 1322, and the transmission gear 142 is rotatable about the transmission shaft 141 under the action of the push-pull rod 1322.

In this embodiment, as shown in FIGs. 4 and 5, the transmission assembly 140 includes a transmission gear 142 located in the middle of the transmission shaft 141, and the push-pull rod 1322 is arranged opposite to the transmission gear 142, such that when the push-pull portion 132 moves laterally, the push-pull rod 1322 acts on teeth 1422 on the transmission gear 142, thereby causing the transmission gear 142 to rotate by a certain angle. Specifically, when the push-pull portion 132 moves from the second position to the first position, the push-pull rod 1322 pulls the teeth 1422, such that the transmission gear 142 rotates counterclockwise (from the angle shown in FIG. 5). In other embodiments of the present application, when the push-pull portion 132 moves from the first position to the second position, the push-pull rod 1322 pushes the teeth 1422, such that the transmission gear 142 rotates clockwise (from the angle shown in FIG. 5). It can be understood that the objectives of the present application are achieved as long as the lateral movement of the push-pull portion 132 can be converted into the unidirectional rotational movement of the transmission gear 142.

In other embodiments of the present application, the transmission assembly 140 includes at least two transmission gears 142, the push-pull portion 132 includes at least one push-pull rod 1322, and the push-pull rod 1322 can act on one or more of the transmission gears 142. In some other embodiments of the present application, the push-pull portion 132 includes at least two push-pull rods 1322, and the at least two push-pull rods 1322 may jointly act on the same transmission gear 142 or may act on different transmission gears 142.

The dose indication device 100 provided in the present application further includes a dose indicator ring 120, as shown in FIG. 4, the dose indicator ring 120 is coaxially arranged with the base 160 and is rotatable about the central axis of the base 160, graduations for displaying count values are provided on the outer periphery of the dose indicator ring 120 (not shown in the figure), and the dose indicator ring 120 is further provided with protruding portions 121 capable of interacting with the transmission gear 142. It can be understood that the position of the dose indication window 112 is set to correspond to the position of the graduations.

In order to limit the position of the dose indicator ring 120, the base 160 is provided with a plurality of dose indicator ring brackets 164. The plurality of dose indicator ring brackets 164 are arranged around the outer periphery or inner wall of the dose indicator ring 120 to avoid misalignment of the dose indicator ring 120.

In this embodiment, as shown in FIG. 5, by means of the protruding portions 121, the rotational movement of the transmission gear 142 in the longitudinal direction may be converted into rotational movement of the dose indicator ring 120 in the horizontal direction around the central axis of the base 160, and then the graduations located on the outer periphery of the dose indicator ring 120 are displayed by the dose indication window 112 to achieve dose indication.

When the fluid container 300 moves up and down within the inhaler 500 together with the dose indication device 100, the actuating portion 131 on the dose indication device 100 interacts with the driving portion 201 located on the housing 200, such that the actuating assembly 130 moves laterally between the first position and the second position. Meanwhile, the push-pull rod 1322 drives the transmission gear 142 to rotate, and then the protruding portions 121 drive the dose indicator ring 120 to rotate, thereby achieving dose indication.

In an embodiment of the present application, the fluid container 300 moves up and down relative to the housing 200, such that the dose indication device 100 is driven to move up and down relative to the housing 200. It can be understood by those skilled in the art that the fluid container 300 and the housing 200 may be that the housing 200 remains stationary while the fluid container 300 moves up and down, or that the housing 200 moves up and down while the fluid container 300 remains stationary, or that both the fluid container 300 and the housing 200 move up and down.

In an embodiment of the present application, a fluid container connecting portion 113 for connecting the fluid container 300 is provided at the end of the outer shell 110 away from the base 160. In an embodiment of the present application, the connection manner is a snap-fit connection. It can be understood that another connection manner, such as a direct connection or an indirect connection, or a fixed connection or a detachable connection, can achieve the objectives of the present application.

In an embodiment of the present application, in order to improve the aesthetics of the device, the dose indication window 112 and the opening 111 on the outer shell 110 are arranged opposite to each other. It can be understood that in other embodiments of the present application, the objectives of the present application can be achieved as long as the dose indication window 112 and the opening 111 do not affect each other.

In some embodiments of the present application, the dose indication device 100 includes at least two actuating assemblies 130. The at least two actuating assemblies 130 are symmetrically arranged relative to the central axis of the dose indication device 100, which is beneficial for the driving portion 201 to provide a balanced acting force on the dose indication device 100 in the actuated state, which is beneficial for improving the driving stability of the dose indication device 100. It can be understood that the number and positions of the openings 111 match the number and positions of the actuating assemblies 130, and the openings 111 do not affect the dose indication window 112.

In a preferred embodiment of the present application, the dose indication device 100 includes two actuating assemblies 130, two openings 111 are correspondingly provided on a side wall of the outer shell 110, and the two openings 111 are located on two sides of the dose indication window 112, respectively. The two actuating assemblies 130 may act on the same transmission assembly 140, or may act on different transmission assemblies 140.

In an embodiment of the present application, in order to make full use of the respective component structures of the dose indication device 100 and improve the aesthetics of the device, when the actuating assembly 130 is at the second position, the actuating portion 131 is configured to completely retract from the opening 111 into the cavity. In other embodiments of the present application, when the actuating assembly 130 is at the second position, the actuating portion 131 still partially protrudes outward from the opening 111, but the part of the actuating portion 131 protruding outward from the opening 111 when the actuating portion is at the second position is reduced compared with the case where the actuating portion is at the first position. It can be understood by those skilled in the art that the objectives of the present application can be achieved as long as the lateral movement of the actuating assembly 130 can be translated into the rotation of the transmission assembly 140 by a certain angle by the push-pull rod 1322.

In an embodiment of the present application, as shown in FIGs. 4 and 5, a stop portion 162 protrudes upward from the side of the base 160 facing the outer shell 110, an elastic assembly 150 is provided between the actuating assembly 130 and the stop portion 162, one end of the elastic assembly 150 abuts against the stop portion 162, and the other end thereof abuts against the actuating assembly 130.

In this embodiment, as shown in FIG. 7, when the actuating assembly 130 is actuated by the driving portion 201, the driving portion 201 applies an acting force to the actuating assembly 130, such that the actuating assembly 130 moves from the first position to the second position against the action of the elastic assembly 150. When the driving portion 201 is away from the actuating assembly 130, an acting force between the driving portion 201 and the actuating assembly 130 continuously decreases, and under the action of the elastic assembly 150, the actuating assembly 130 returns from the second position to the first position. As shown in FIG. 6, when the actuating assembly 130 is at the first position, there is no interaction force between the driving portion 201 and the actuating assembly 130.

In an embodiment of the present application, as shown in FIGs. 4 and 5, the elastic assembly 150 is a spring, one end of the spring abuts against the stop portion 162, and the other end thereof abuts against the side of the actuating portion away from the housing 200.

In an embodiment of the present application, the driving portion 201 is an inward protruding structure provided on an inner wall of the housing 200.

In an embodiment of the present application, as shown in FIGs. 1-2 and 6-7, in an initial state, the driving portion 201 is disengaged from the actuating assembly 130, and the actuating assembly 130 is at the first position. When the dose indication device 100 moves downwards along with the fluid container 300 relative to the housing 200, the protruding structure abuts against the actuating portion 131 and provides an acting force to move the actuating portion 131 from the first position to the second position, and the dose indication device 100 is in an actuated state. When the dose indication device 100 moves upwards relative to the housing 200, the protruding structure is disengaged from the actuating portion 131, and under the action of the elastic assembly 150, the actuating portion 131 returns from the second position to the first position, thereby achieving one cycle.

Similarly, in another embodiment of the present application, in an initial state, the driving portion 201 abuts against the actuating assembly 130 and provides an acting force such that the actuating assembly 130 is at the second position. When the dose indication device 100 moves downwards along with the fluid container 300 relative to the housing 200, the protruding structure is disengaged from the actuating portion 131, and under the action of the elastic assembly 150, the actuating portion 131 moves from the second position to the first position. When the dose indication device 100 moves upwards relative to the housing 200, the protruding structure abuts against the actuating portion 131 again, such that the actuating portion 131 returns from the first position to the second position, thereby achieving one cycle.

It can be understood that, regardless of whether the actuating assembly 130 is at the first position or the second position in the initial state, the objectives of the present application can be achieved as long as the actuating assembly can reciprocate laterally between the first position and the second position.

In some embodiments of the present application, the driving portion 201 is a separate component that is detachably or fixedly connected to the housing 200. When the dose indication device 100 moves relative to the housing 200, the driving portion 201 enables the actuating assembly 130 to move laterally between the first position and the second position.

In this embodiment, designing the driving portion 201 as a separate component can further increase the versatility of the dose indication device 100, especially for adapting to fluid containers 300 of different widths or housings 200 of different inner diameters. In the production process, the fluid container 300 and the dose indication device 100 of the same specification may be adapted to the housings 200 of different inner diameters by only adjusting the dimensions of the driving portion 201, or the fluid container 300 and the dose indication device 100 of different diameters may be adapted to the housing 200 of the same specification, which improves the flexibility of mating between components and reduces the production cost.

In some embodiments of the present application, to simplify the structure, the driving portion 201 is a part of the housing 200, and when the fluid container 300 moves relative to the housing 200 together with the dose indication device 100, the actuating portion 131 of the actuating assembly 130 comes into contact with the driving portion 201, and the driving portion 201 actuates the actuating portion 131, such that the actuating assembly 130 moves laterally between the first position and the second position.

In an embodiment of the present application, the inner wall of the lower end of the housing 200 gradually contracts inward along the central axis of the housing 200 to form the driving portion 201, when the actuating assembly 130 is located at the first position, the actuating portion 131 is not in contact with the driving portion 201, and when the actuating assembly 130 moves from the first position to the second position, the actuating portion 131 abuts against the driving portion 201, and an acting force between the inner wall and the actuating portion 131 causes the actuating portion 131 to at least partially retract into the outer shell 110.

In this embodiment, at least part of the inner wall of the lower end of the housing 200 gradually contracts to form the driving portion 201, and when the dose indication device 100 is mounted, the actuating portion 131 needs to correspond to the driving portion 201.

In a preferred embodiment of the present application, as shown in FIGs. 1-3, the inner wall of the lower end of the housing 200 gradually contracts circumferentially inward along the central axis of the housing 200 to form the driving portion 201. In this way, the component structure is simplified, and moreover, since the horizontal cross-section of the inner wall of the lower end of the housing 200 is substantially circular, when the dose indication device 100 is mounted on the fluid container 300, the actuating portion 131 can be actuated by the driving portion 201 as long as the actuating portion 131 faces the inner wall of the housing 200, regardless of the specific direction, which is beneficial for simplifying the assembly of the dose indication device 100.

In this embodiment, as shown in FIG. 1, in the initial state, the fluid container 300 and the dose indication device 100 are at the top position, the inner diameter of the housing 200 is greater than the maximum width D1 of the dose indication device, and the actuating portion 131 is at the first position; when the dose indication device 100 moves downward along with the fluid container 300, the inner diameter of the housing 200 gradually decreases, and when the inner diameter of the housing 200 is equal to the maximum width D1 of the dose indication device, the driving portion 201 abuts against the actuating portion 131, and the actuating portion 131 starts to be actuated; as the dose indication device 100 continues to move downward, the acting force between the driving portion 201 and the actuating portion 131 gradually pushes the actuating portion 131 in a direction away from the housing 200 until the part of the actuating portion 131 protruding outward from the opening 111 is minimized. At this point, the inner diameter of the housing 200 is equal to the minimum width D2 of the dose indication device, and as shown in FIG. 2, the actuating portion 131 is at the second position. When the fluid container 300 and the dose indication device 100 move upward relative to the housing 200, the inner diameter of the housing 200 gradually increases, and the acting force between the driving portion 201 and the actuating portion 131 gradually decreases until the driving portion 201 and the actuating portion 131 are completely disengaged. Under the action of the elastic assembly 150, the actuating portion 131 returns from the second position to the first position, thereby achieving one cycle.

In some embodiments of the present application, in order to reduce the movement resistance, the contact surfaces between the actuating portion 131 and the driving portion 201 are configured as matched smooth curved surfaces. In some embodiments of the present application, at least two rib structures are provided on the inner wall of the housing 200, guide grooves for allowing the actuating portion 131 to move are formed between the rib structures, and the guide grooves are offset from top to bottom in a direction toward the central axis of the housing 200.

In this embodiment, when the dose indication device 100 moves downward together with the fluid container 300, the actuating portion 131 moves in the guide grooves, and since the guide grooves are offset from top to bottom in the direction toward the central axis of the housing 200, the bottoms of the guide grooves serve as the driving portion 201, such that the actuating portion 131 moves laterally between the first position and the second position.

In some embodiments of the present application, 2-40 rib structures are evenly arranged on the inner wall of the housing 200; in some embodiments of the present application, 2-30 rib structures are evenly arranged on the inner wall of the housing 200; in some embodiments of the present application, 10-30 rib structures are evenly arranged on the inner wall of the housing 200; in some preferred embodiments of the present application, 20-30 rib structures are evenly arranged on the inner wall of the housing 200. In some embodiments of the present application, the guide grooves provided between the rib structures are offset from top to bottom by the same angle in the direction toward the central axis of the housing 200, which is beneficial for the assembly of the dose indication device 100. In some other embodiments of the present application, the guide grooves provided between the rib structures are offset from top to bottom by different angles in a direction toward the central axis of the housing 200, which is beneficial to improving the versatility of the device, and thus the device can adapt to fluid containers 300 of different widths and dose indication devices 100 of different widths.

In some embodiments of the present application, in order to reduce the movement resistance, the bottom of the guide groove is a smooth curved surface, and the contact surfaces between the actuating portion 131 and the guide groove are also configured as matched smooth curved surfaces.

In some embodiments of the present application, the dose indication device 100 further includes a transmission assembly bracket 161 fixed to the base 160, and the transmission assembly 140 is rotatably arranged in the transmission assembly bracket 161. The above design enables the transmission shaft 141 to rotate only on the transmission assembly bracket 161, while preventing movement in other directions, and the transmission shaft 141 is parallel to the push-pull rod 1322.

In an embodiment of the present application, as shown in FIGs. 5 and 10-12, the protruding portions 121 are located at the top of the dose indicator ring 120 and axially extend in the cavity, the transmission gear 142 includes a helical groove 1421 and radially protruding teeth 1422, and during rotation of the transmission gear 142, at least one protruding portion 121 engages in the helical groove 1421 all the time.

In this embodiment, a worm drive is formed between the transmission gear 142 and the dose indicator ring 120 through the helical groove 1421 and the protruding portions 121, such that the rotational movement of the transmission gear 142 in the vertical direction is converted into the rotational movement of the dose indicator ring 120 in the horizontal direction. When the transmission gear 142 rotates in the vertical direction, the protruding portions 121 move along the helical groove 1421, causing the dose indicator ring 120 to rotate in the horizontal direction. Moreover, when the previous protruding portion 121 moves out of the helical groove 1421, the next protruding portion 121 enters the helical groove 1421, such that at least one protruding portion 121 engages in the helical groove 1421 all the time, thereby ensuring the continuity of the rotational movement of the dose indicator ring 120 in the horizontal direction.

It can be understood that in other embodiments of the present application, the objectives of the present application can be achieved as long as other driving manners can convert the rotational movement of the transmission gear 142 in the vertical direction into the rotational movement of the dose indicator ring 120 in the horizontal direction.

In an embodiment of the present application, as shown in FIGs. 5-7, the push-pull rod 1322 acts on the teeth 1422 to drive the transmission gear 142 to rotate.

In this embodiment, the push-pull rod 1322 and the teeth 1422 are arranged opposite to each other, such that when the push-pull portion 132 moves laterally, the push-pull rod 1322 acts on the teeth 1422 on the transmission gear 142, thereby causing the transmission gear 142 to rotate by a certain angle. Specifically, when the push-pull portion 132 moves from the first position to the second position, the push-pull rod 1322 does not act on the teeth 1422, or a contact portion of the push-pull rod 1322 and/or the teeth 1422 is elastically deformed, but the transmission gear 142 does not rotate; when the push-pull portion 132 moves from the second position to the first position, the push-pull rod 1322 pulls the teeth 1422, such that the transmission gear 142 rotates counterclockwise (from the angle shown in FIG. 5). In other embodiments of the present application, when the push-pull portion 132 moves from the first position to the second position, the push-pull rod 1322 pushes the teeth 1422 such that the transmission gear 142 rotates clockwise (from the angle shown in FIG. 5); when the push-pull portion 132 moves from the second position to the first position, the transmission gear 142 does not rotate. It can be understood that the objectives of the present application are achieved as long as the lateral movement of the push-pull portion 132 can be converted into the unidirectional rotational movement of the transmission gear 142.

In order to ensure accurate counting of the dose indication device 100 and prevent the transmission gear 142 from rotating in reverse, in an embodiment of the present application, the dose indication device 100 further includes a lock pawl 163 for preventing the transmission gear 142 from rotating in reverse, the lock pawl 163 being arranged within the cavity and extending toward the transmission gear 142.

In this embodiment, as shown in FIGs. 5-9, the lock pawl 163 is arranged on the base 160, and the lock pawl 163 acts on the teeth 1422 on the transmission gear 142 to ensure that the transmission gear 142 rotates in one direction all the time.

In a preferred embodiment of the present application, the stop portion 162 and the lock pawl 163 are the same component, and the component has the functions of both the stop portion 162 and the lock pawl 163, which can further simplify the structure of the dose indication device 100 and reduce the production cost.

Although the above-described dose indication device 100 can inform the user of the usage of the fluid container 300, the user may forget to observe the usage of the fluid container. When the fluid in the fluid container 300 reaches or exceeds the predetermined number of uses, especially when the fluid is nearly depleted or has been depleted, if the user continues to use the fluid container 300 without timely replacing the fluid container 300 with a new one, it is likely that the ejected dose is different from the predetermined dose, which may cause a risk of medication.

In order to avoid the above problems, in some embodiments of the present application, the dose indication device 100 further includes a locking mechanism configured to prevent the actuating portion 131 from moving laterally between the first position and the second position and/or to prevent the dose indicator ring 120 from further rotating after a predetermined number of uses of the fluid container 300.

In an embodiment of the present application, the locking mechanism includes a first locking portion 133 located on the actuating assembly 130 and a second locking portion 122 located on the dose indicator ring 120, the second locking portion 122 engaging with the first locking portion 133 after the predetermined number of uses of the fluid container 300, so as to prevent the actuating assembly 130 from moving laterally between the first position and the second position.

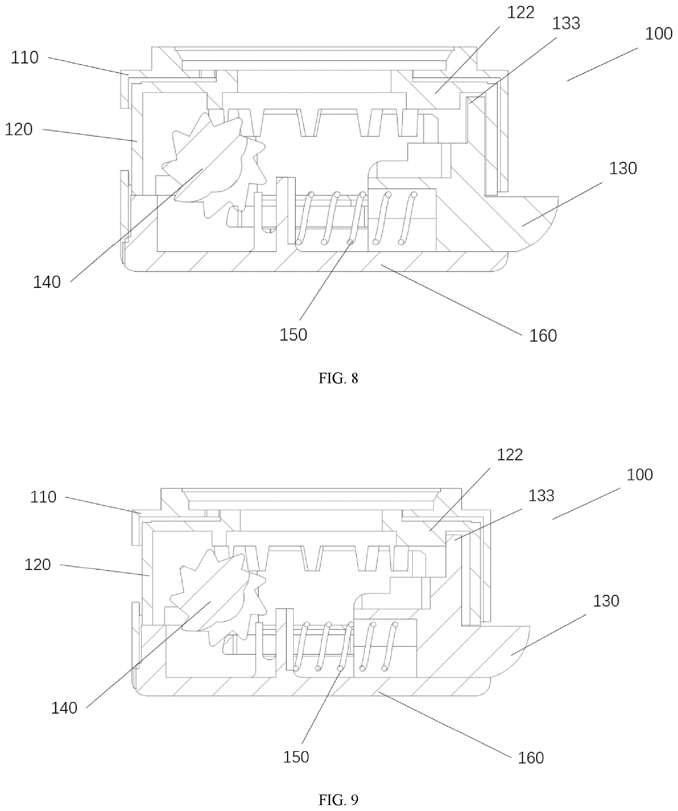

In this embodiment, as shown in FIGs. 8 and 9, after the predetermined number of uses of the fluid container 300, the second locking portion 122 located on the dose indicator ring 120 rotates to a corresponding position of the first locking portion 133 on the actuating assembly 130, thereby preventing the actuating assembly 130 from moving laterally. In this case, even if the driving portion 201 acts on the actuating portion 131, the actuating assembly 130 cannot move laterally from the first position to the second position.

It can be understood by those skilled in the art that the objectives of the present application can be achieved as long as the second locking portion 122 can rotate to the corresponding position of the first locking portion 133 after the predetermined number of uses of the fluid container 300 to prevent the actuating assembly 130 from moving laterally between the first position and the second position. In other embodiments of the present application, when the second locking portion 122 can rotate to the corresponding position of the first locking portion 133, the objectives of the present application can also be achieved by preventing the actuating assembly 130 from moving laterally from the second position to the first position.

In a preferred embodiment of the present application, the first locking portion 133 is located in the cavity and extends toward the top of the dose indicator ring 120, the second locking portion 122 is located at the top of the dose indicator ring 120 and extends toward the base 160, and after the predetermined number of uses of the fluid container 300, the second locking portion 122 engages with the first locking portion 133, so as to prevent the actuating assembly 130 from moving laterally between the first position and the second position. In other embodiments of the present application, the first locking portion 133 and the second locking portion 122 may be located at other positions, such as on the push-pull portion 132, on the side wall of the dose indicator ring 120, etc., as long as the second locking portion 122 engages with the first locking portion 133 after the predetermined number of uses of the fluid container 300 to prevent the actuating assembly 130 from moving laterally between the first position and the second position. In this way, the objectives of the present application can be achieved.

In an embodiment of the present application, the locking mechanism prevents the actuating assembly 130 from moving laterally after the predetermined number of uses of the fluid container 300.

In this embodiment, after the predetermined number of uses of the fluid container 300, the second locking portion 122 rotates to a position where the second locking portion abuts against the first locking portion 133, preventing any lateral movement of the actuating assembly 130.

In another preferred embodiment of the present application, as shown in FIGs. 8 and 9, the second locking portion 122 is located on a path of lateral movement of the first locking portion 133, thereby preventing the actuating assembly 130 from moving laterally from the first position to the second position.

In this embodiment, the actuating assembly 130 may partially move laterally, but cannot completely move laterally from the first position to the second position. Under the action of the driving portion 201, the actuating portion 131 retracts into the outer shell 110, but cannot retract back to the second position. The push-pull rod 1322 cannot rotate the transmission gear 142 by a certain angle, and thus the dose indicator ring 120 cannot rotate in the horizontal direction either, thereby achieving the locking of the dose indication device 100.

In this embodiment, when the second locking portion 122 abuts against the first locking portion 133, the actuating portion 131 also provides an opposite acting force to the driving portion 201, so as to prevent the fluid container 300 and the dose indication device 100 from further moving downward relative to the housing 200, thereby locking the inhaler 500.

In another embodiment of the present application, the locking mechanism prevents the actuating assembly 130 from moving laterally from the second position to the first position after the predetermined number of uses of the fluid container 300. The driving portion 201 cannot effectively actuate the actuating portion 131 again to achieve the locking of the dose indication device 100.

In order to prevent the dose indication device 100 from being erroneously actuated during production, assembly or transport of the dose indication device 100, or prior to the mounting of the fluid container 300 onto the inhaler 500 for nebulized drug delivery, leading to inaccurate counts and displays, the dose indication device 100 further includes an anti-inadvertent actuation mechanism 170 which prevents the dose indication device 100 from being inadvertently actuated prior to use of the fluid container 300.

In an embodiment of the present application, the anti-inadvertent actuation mechanism 170 is detachably connected to the actuating portion 131, and when the anti-inadvertent actuation mechanism 170 is disengaged from the actuating portion 131, the actuating portion 131 is in an actuatable state.

In this embodiment, when the anti-inadvertent actuation mechanism 170 is connected to the actuating portion 131, the actuating portion 131 is in a non-actuatable state, and even if the driving portion 201 acts on the actuating portion 131, the actuating portion 131 cannot move laterally. Only when the anti-inadvertent actuation mechanism 170 is removed from the actuating portion 131, can the actuating portion 131 be normally actuated.

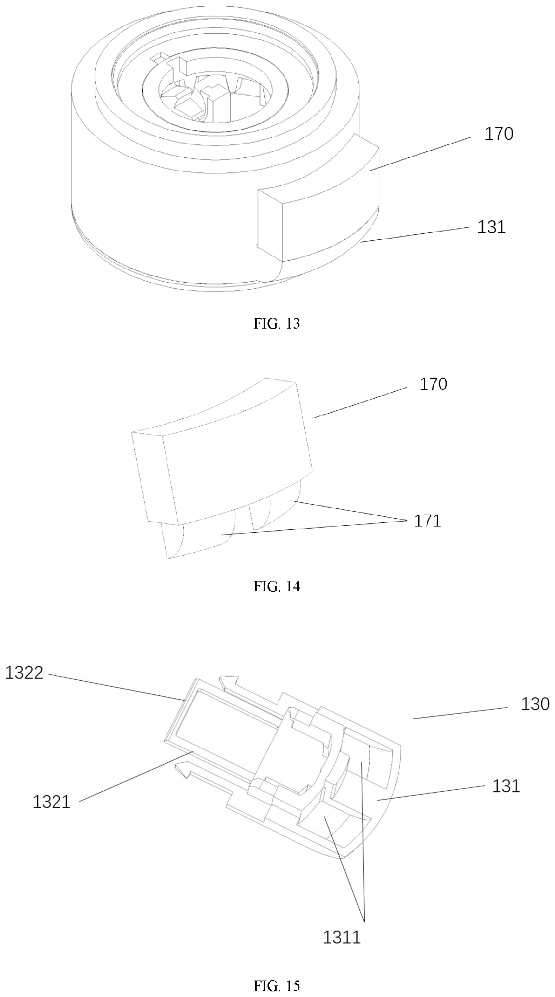

In a preferred embodiment of the present application, as shown in FIGs. 13-15, the anti-inadvertent actuation mechanism 170 includes protrusions 171, grooves 1311 matched with the protrusions 171 are provided in a part of the actuating portion 131 located outside the cavity, and prior to use of the fluid container 300, the anti-inadvertent actuation mechanism 170 is connected to the grooves 1311 through the protrusions 171, such that the actuating portion 131 is in a non-actuatable state.

In this embodiment, a latch structure is formed between the anti-inadvertent actuation mechanism 170 and the actuating portion 131. It can be understood that in other embodiments of the present application, the objectives of the present application can also be achieved by means of other detachable connection manners as long as the actuating portion 131 can be prevented from moving laterally.

In a preferred embodiment of the present application, the anti-inadvertent actuation mechanism 170 includes two protrusions 171, and the actuating portion 131 is correspondingly provided with two grooves 1311. This design can prevent the anti-inadvertent actuation mechanism 170 from being inadvertently disengaged from the actuating portion 131 and improve the stability of the connection between the anti-inadvertent actuation mechanism 170 and the actuating portion 131.

The foregoing descriptions are merely specific implementations of the present application, but are not intended to limit the scope of the present application. Any equivalent variations, modifications, or combinations made by those skilled in the art without departing from the spirit and principles of the present application shall fall within the scope of protection of the present application.

## Claims

1. A dose indication device for an inhaler, the inhaler comprising: a housing for receiving a fluid container and a nebulising assembly for nebulising a fluid, the fluid container being movable relative to the housing, and the housing comprising a driving portion for actuating the dose indication device; the dose indication device comprising:
an outer shell, through which the dose indication device is connected to the fluid container, and a dose indication window and an opening being provided on a side wall of the outer shell;
a base, connected to the outer shell, and a cavity being provided between the outer shell and the base;
an actuating assembly, slidably connected to the base, under actuation of the driving portion, the actuating assembly being laterally movable relative to the base between a first position and a second position, the actuating assembly comprising an actuating portion and a push-pull portion connected to each other, the actuating portion penetrating through the opening, when the actuating assembly is at the first position, the actuating portion at least partially protruding outward from the opening, and when the actuating assembly is at the second position, the actuating portion at least partially retracting into the cavity; the push-pull portion comprising a push-pull rod and being located in the cavity;
a transmission assembly, located in the cavity and comprising a transmission shaft and a transmission gear located on the transmission shaft, the transmission gear being arranged opposite to the push-pull rod, and the transmission gear being rotatable about the transmission shaft under an action of the push-pull rod; and
a dose indicator ring, coaxially arranged with the base and rotatable about a central axis of the base, graduations for displaying count values being provided on an outer periphery of the dose indicator ring, and the dose indicator ring being further provided with protruding portions capable of interacting with the transmission gear; and
when the actuating assembly moves between the first position and the second position, the push-pull rod driving the transmission gear to rotate, which in turn causes the dose indicator ring to rotate via the protruding portions, thereby achieving dose indication.

2. The dose indication device according to claim 1, comprising at least two actuating assemblies, wherein the at least two actuating assemblies are symmetrically arranged relative to a central axis of the outer shell, and a number and positions of the openings match a number and positions of the actuating assemblies.

3. The dose indication device according to claim 1, wherein the push-pull portion further comprises a connecting rod, and the push-pull rod is located at end parts of the connecting rod and is fixedly connected to the actuating portion through the other end parts of the connecting rod.

4. The dose indication device according to claim 1, wherein a stop portion is provided on a side of the base facing the outer shell, an elastic assembly is provided between the actuating assembly and the stop portion, one end of the elastic assembly abuts against the stop portion, and the other end thereof abuts against the actuating assembly.

5. The dose indication device according to any one of claims 1 to 4, wherein the driving portion is an inward protruding structure provided on an inner wall of the housing.

6. The dose indication device according to claim 5, wherein the driving portion is a separate component detachably or fixedly connected to the housing, and when the dose indication device moves relative to the housing, the driving portion enables the actuating assembly to move laterally between the first position and the second position; or
the driving portion is a part of the housing, and when the fluid container moves relative to the housing together with the dose indication device, the actuating portion of the actuating assembly comes into contact with the driving portion, and the driving portion actuates the actuating portion, such that the actuating assembly moves laterally between the first position and the second position.

7. The dose indication device according to claim 5, wherein an inner wall of a lower end of the housing gradually contracts inward along a central axis of the housing to form the driving portion, when the actuating assembly is at the first position, the actuating portion does not abut against the driving portion, and when the actuating assembly moves from the first position to the second position, the actuating portion abuts against the driving portion, and an acting force between the driving portion and the actuating portion causes the actuating portion to at least partially retract into the outer shell.

8. The dose indication device according to claim 5, wherein at least two rib structures are provided on the inner wall of the housing, guide grooves for allowing the actuating portion to move are formed between the rib structures, the guide grooves are offset from top to bottom in a direction toward a central axis of the housing, and 2-40 rib structures are evenly arranged on the inner wall of the housing.

9. The dose indication device according to claim 1, wherein the dose indication device further comprises a transmission assembly bracket fixed to the base, and the transmission assembly is rotatably arranged in the transmission assembly bracket.

10. The dose indication device according to claim 1, wherein the protruding portions are located at a top of the dose indicator ring and axially extend toward the transmission gear, the transmission gear comprises a helical groove and radially protruding teeth, and during rotation of the transmission gear, at least one protruding portion engages in the helical groove all the time.

11. The dose indication device according to claim 10, wherein the push-pull rod acts on the teeth to drive the transmission gear to rotate.

12. The dose indication device according to claim 1, further comprising a lock pawl for preventing the transmission gear from rotating in reverse, the lock pawl being arranged within the cavity and extending toward the transmission gear.

13. The dose indication device according to claim 1, further comprising a locking mechanism configured to prevent the actuating portion from moving laterally between the first position and the second position and/or to prevent the dose indicator ring from further rotating after a predetermined number of uses of the fluid container.

14. The dose indication device according to claim 13, wherein the locking mechanism comprises a first locking portion located on the actuating assembly and a second locking portion located on the dose indicator ring, the second locking portion engaging with the first locking portion after the predetermined number of uses of the fluid container, so as to prevent the actuating assembly from moving laterally between the first position and the second position; or the locking mechanism comprises a first locking portion located in the cavity and extending toward a top of the dose indicator ring, and a second locking portion located at the top of the dose indicator ring and extending toward the base, the second locking portion engaging with the first locking portion after the predetermined number of uses of the fluid container, so as to prevent the actuating assembly from moving laterally between the first position and the second position.

15. The dose indication device according to claim 13, wherein the locking mechanism prevents the actuating assembly from moving laterally after the predetermined number of uses of the fluid container, or
the locking mechanism prevents the actuating assembly from moving laterally from the second position to the first position.

16. The dose indication device according to claim 1, further comprising an anti-inadvertent actuation mechanism for preventing the dose indication device from being inadvertently actuated prior to use of the fluid container.

17. A fluid container for an inhaler, a bottom of the fluid container being connected to the dose indication device according to any one of claims 1 to 16.

18. The fluid container according to claim 17, the fluid container being fixedly connected to the dose indication device, and when the fluid container needs to be replaced, the fluid container and the dose indication device being replaced together.

19. An inhaler, a housing of the inhaler receiving the fluid container according to claim 17 or 18.

20. A method for counting and/or indicating the number of times the fluid container in the inhaler has been used or the remaining number of uses by using the dose indication device according to any one of claims 1 to 19.
